# DEMANDE DE BREVET EUROPEEN

(11) **EP 2 008 665 A1**
(43) Date de publication de la demande: **31.12.2008**
(21) Numéro de dépôt: 07290783.5
(22) Date de dépôt: 25.06.2007
(51) Int. Cl.: A61K 41/00, A61P 35/00, A61K 47/26

(54) **Formulation injectable pour thérapie photodynamique**

(71) Demandeur: Steba Biotech N.V., 2585 GB Den Haag (NL)
(72) Inventeur: Eren, Doron, Netaim 76870 Israel (IL); Benjamin, Eran, J., Rehovot 76468 (IL); Neimann, Karine, Nes-Ziona 74037 (IL); Ben-Ami, Goni, Rehovot 76276 (IL)
(74) Mandataire: L'Helgoualch, Jean

(57) **Abrégé**

L'invention concerne une formulation pour administration par injection, comprenant un agent photosensibilisant utilisable en thérapie photodynamique ou en diagnostic représentée par la formule générale (II) suivante : 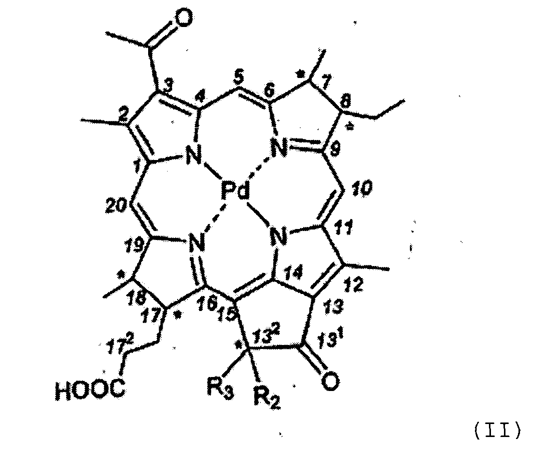 dans laquelle R₂ est un hydrogène, un hydroxyle ou un groupe -COOR₄ dans lequel R₄ est un hydrogène, un alkyle en C₁-C₁₂ ou un cycloalkyle en C₃-C₁₂, et R₃ est un hydrogène, un hydroxyle ou un alkyle ou alcoxy en C₁-C₁₂, ou un de ses sels de métaux alcalins ou alcalino-terreux,
en combinaison avec un agent tensioactif et du mannitol.

Cette formulation limite le taux d'agrégation de l'agent photosensibilisant.

## Description

La présente invention concerne une nouvelle formulation galénique pour administration par injection, et plus particulièrement une nouvelle formulation adaptée à l'administration d'un agent photosensibilisant pour thérapie photodynamique à base de bactériochlorophylle associée à un support ou véhicule pharmaceutiquement acceptable, ainsi qu'un procédé pour la préparation d'une telle formulation.

De nombreuses études ont montré que diverses substances possédant des propriétés photosensibilisantes peuvent être utilisées en thérapie photodynamique (PDT) pour le traitement et le diagnostic de certaines maladies telles que divers types de cancers.

Les techniques de thérapies photodynamiques sont aujourd'hui indiquées ou préconisées dans divers domaines, par exemple en dermatologie pour le traitement de kératoses actiniques, en ophtalmologie pour le traitement de la dégénérescence maculaire liée à l'âge, ainsi qu'en oncologie pour le traitement de tumeurs cérébrales, de tumeurs de l'appareil digestif, du poumon ou de la prostate.

La technique de PDT consiste à administrer la substance photosensibilisante, qui se fixe préférentiellement dans les tissus sur les cellules tumorales, selon les photosensibilisants utilisés, et à illuminer ensuite les tissus au moyen d'une source lumineuse de longueur d'onde appropriée qui est susceptible d'activer la substance photosensibilisante. Lorsqu'elle a été activée par les photons de la lumière monochromatique (laser) ou polychromatique (lampe), la substance photosensibilisante, qui est initialement non toxique, passe de son état d'énergie de base à un état d'énergie supérieur, dénommé "singulet excité", et peut libérer in situ de l'oxygène singulet ou des radicaux libres (espèces radicalaires oxygénées) qui sont extrêmement réactifs et oxydent les tissus immédiatement voisins, provoquant la mort des cellules, et en particulier des cellules cancéreuses. On peut se référer notamment à K.R. Weishaupt et al., Cancer Research, p. 2326-2329 (1976) et T.J. Dougherty et al. "Photodynamic therapy (PDT) and clinical applications", Biomedical Photonics Handbook. CRC Press LLC. 38: 1-38 (2003). De préférence, les espèces radicalaires oxygénées ainsi produites ont une faible diffusion et une très courte durée de vie si bien que leur effet toxique est très localisé.

Les premières substances étudiées dans ce domaine sont des dérivés d'hématoporphyrine tels que le porfimère sodique (Photofrin®) qui est un mélange d'esters et d'éthers de dihématoporphyrine. Le Photofrin® présente cependant l'inconvénient de devoir être administré par voie intraveineuse deux jours avant le traitement d'activation par la source lumineuse, et son accumulation dans l'organisme avant élimination entraîne une photosensibilisation cutanée pendant 4 à 8 semaines; de plus, à la longueur d'onde utilisée (630 nm) la pénétration de la lumière est limitée à quelques millimètres, et l'utilisation de cet agent photosensibilisant est donc limitée aux tumeurs de petite dimension, localisées en surface des tissus. On peut se référer par exemple à Doiron et al. "Porphyrins in Tumor Phototherapy", Plenum Press NY, pp. 281-291 (1984). On a donc recherché des agents photosensibilisants susceptibles d'être activés à des longueurs d'ondes plus grandes et pouvant être éliminés rapidement.

De nombreux autres dérivés ont été proposés pour les traitements par PDT, par exemple des dérivés de benzoporphyrine qui ont pour avantage d'être activés à des longueurs d'ondes plus élevées que le Photofrin (env. 690 nm), ce qui a pour conséquence une meilleure pénétration des rayons lumineux dans les tissus. De plus les dérivés de benzoporphyrine sont éliminés plus rapidement de l'organisme et présentent moins d'effets secondaires que le Photofrin®, en particulier une moindre photosensibilisation cutanée. Cependant, ils sont très peu solubles en milieux aqueux et leur administration par injection parentérale est difficile. Pour pallier ces inconvénients, le brevet US 5.289.378 propose d'encapsuler les benzoporphyrines dans des liposomes. Un médicament à base d'une benzoporphyrine de ce type, le verteporfin (Visudyne®), est utilisé pour le traitement de la dégénérescence maculaire liée à l'âge.

Les recherches sur les substances photosensibilisantes ont montré que des dérivés de bactériochlorophylle possèdent d'intéressantes propriétés utilisables dans les techniques de thérapie photodynamique.

Des dérivés de bactériochlorophylle de formule générale (I) suivante ont été décrits dans le brevet européen EP 584.552 :

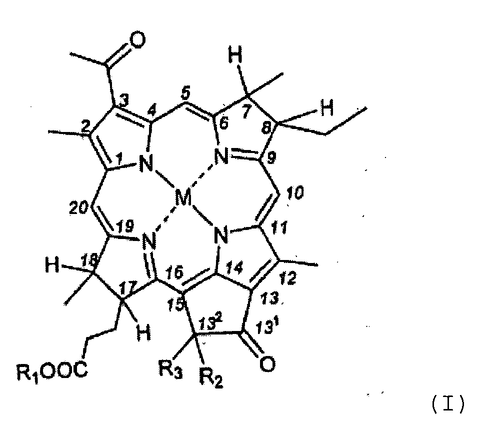

De préférence, l'atome métallique M dans cette formule est un atome de magnésium, le substituant R₁ est un groupe phytyle, R₂ est un hydrogène et R₃ un groupe acétoxy. Le dérivé correspondant est alors la bactériochlorophylle (Bchl). La formule dans laquelle R₁ est un hydrogène est celle du bactériochlorophyllide (Bchlide). Le bactériophéophorbide (Bphéide) est représenté par la même formule que le bactériochlorophyllide en remplaçant l'atome de magnésium par deux atomes d'hydrogène.

Le brevet EP 1.137.411 décrit des dérivés de bactériochlorophylle à substituant palladium présentant une solubilité améliorée, et plus particulièrement des dérivés représentés par la formule générale (II) suivante :

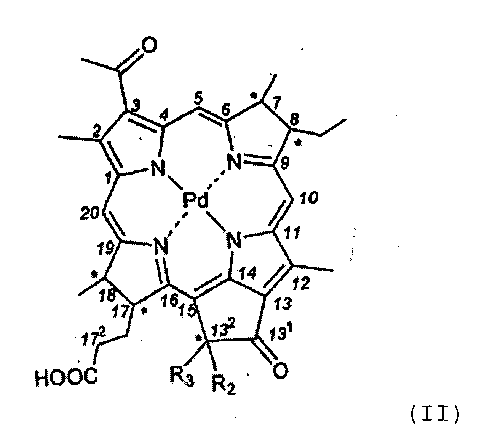

Dans cette formule, où les carbones asymétriques sont repérés par un "*", R₂ est un atome d'hydrogène, un groupe hydroxyle ou un groupe -COOR₄ dans lequel R₄ est un atome d'hydrogène, un groupe alkyle de 1 à 12 atomes de carbone ou un groupe cycloalkyle de 3 à 12 atomes de carbone, et R₃ est un atome d'hydrogène, un groupe hydroxyle ou un groupe alkyle ou alcoxy de 1 à 12 atomes de carbone. De préférence, R₂ est un groupe -COO-alkyle, plus particulièrement un groupe -COOCH₃, et R₃ est un atome d'hydrogène.

Les dérivés de bactériochlorophylle ci-dessus présentent l'avantage d'une photoactivation à une longueur d'onde d'environ 750 à 770 nm, c'est-à-dire bien plus élevée que le Photofrin® et il en résulte une meilleure pénétration dans les tissus de l'organisme. Ainsi, la palladium bactériochlorophylle-a, dont la dénomination chimique est 7,8,17,18-tetra-hydroporphyrine penta-cyclique, comportant un 5ème isocycle et un atome central de palladium, possède des propriétés intéressantes, notamment un coefficient d'extinction important à des longueurs d'ondes élevées (λₘₐₓ=762 nm, ε=1.2×105 M⁻¹cm⁻¹).

Le palladium-bactériophéophorbide-a, ou Pd-bactériophéophorbide-a, encore dénommé Pd-Bphéide-a, décrit dans le brevet EP 1.137.411 et représenté par la formule générale ci-dessus où R₂ est un groupe -COOCH₃ et R₃ est un atome d'hydrogène, est photoactivé à 760 nm, autorisant une bonne pénétration de la lumière dans les tissus, mais il présente un caractère lipophile et une tendance à s'agréger en milieu aqueux, ce qui limite ses possibilités d'utilisation dans des compositions pour administration par voie parentérale.

Le brevet EP 1.137.411 précité indique que pour son administration par voie systémique il est nécessaire de lui ajouter un agent tensioactif en une quantité suffisante pour le solubiliser, par exemple du Cremophor ELP®, qui augmente la solubilité. Cet agent tensioactif à base d'huile de ricin polyoxyéthylénée est utilisé dans des compositions pharmaceutiques en raison de sa capacité à faciliter la solubilisation de principes actifs lipophiles. Cependant, des quantités importantes de Cremophor (20 à 50%) sont nécessaires pour solubiliser le principe actif, et une utilisation à des concentrations aussi élevées a pour inconvénient de provoquer des réactions anaphylactoïdes chez les patients. En pratique, des concentrations de Cremophor supérieures à environ 5% dans des formulations injectables provoquent plus souvent des réactions allergiques. D'autres agents tensioactifs solubilisants connus tels que le propylène glycol, le Solutol®, les lécithines de soja, le Polysorbate 80, etc, n'ont pas donné de résultat satisfaisant.

La demande WO 2003009842 propose de résoudre ce problème au moyen d'une formulation contenant une faible quantité de cosolvant non aqueux choisi parmi le propylène glycol et un mélange alcool benzylique / éthanol dans un rapport allant de 15:85 à 25:75. Il devient alors possible de réduire la quantité d'agent tensioactif, par exemple le Cremophor, et de limiter l'agrégation de l'agent photosensibilisant dans une proportion d'environ 40 à 50% en poids par rapport au poids total de la formulation, et même 20% dans un cas isolé.

Toutefois, les essais effectués ont montré que cette formulation, bien qu'elle réduise l'agrégation de l'agent photosensibilisant, n'est pas totalement satisfaisante car la formation d'agrégats du principe actif dans la formulation a pour effet non seulement de diminuer l'activité photosensibilisante mais aussi, le cas échéant, d'induire une hypotension et d'affecter le foie in vivo.

Aussi, en raison de l'intérêt majeur que présente le Bphéide-a et les dérivés similaires de bactériochlorophylle mentionnés ci-dessus, pour les traitements en thérapie photodynamique, il est important de pouvoir mettre au point une formulation préservant l'efficacité de la substance photosensibilisante et permettant son administration par voie parentérale, de préférence intraveineuse, en évitant les effets secondaires mentionnés ci-dessus. Il est donc souhaitable de pouvoir disposer d'une formulation pour administration par voie parentérale, de préférence par injection, qui comprend un taux d'agrégation d'agent photosensibilisant aussi faible que possible, c'est-à-dire un taux de monomère aussi élevé que possible.

Les expérimentations effectuées sur les composés photosensibilisants de formule (II) ci-dessus ont montré qu'ils sont instables en milieu alcalin, avec notamment une épimérisation en position 13², ce qui constitue un inconvénient pour la mise sous forme pharmaceutique.

Ainsi la solubilisation par salification des composés photosensibilisants entraîne un problème technique lié à l'instabilité, qui interdit l'utilisation des sels solubles. Le problème de la solubilisation ne peut donc pas être résolu par la technique de salification.

La présente invention a donc pour objet une formulation pour administration par injection, de préférence par injection intraveineuse, comprenant une substance photosensibilisante utilisable en thérapie photodynamique ou en diagnostic.

La présente invention a plus particulièrement pour objet une formulation pour administration par injection intraveineuse, comprenant une substance photosensibilisante utilisable en thérapie photodynamique ou en diagnostic, notamment en urologie et en ophtalmologie, plus particulièrement pour le diagnostic et le traitement de cancers tels que le cancer de la prostate, de l'hypertrophie bénigne de la prostate et de la dégénérescence maculaire liée à l'âge, représentée par la formule générale (II) suivante : 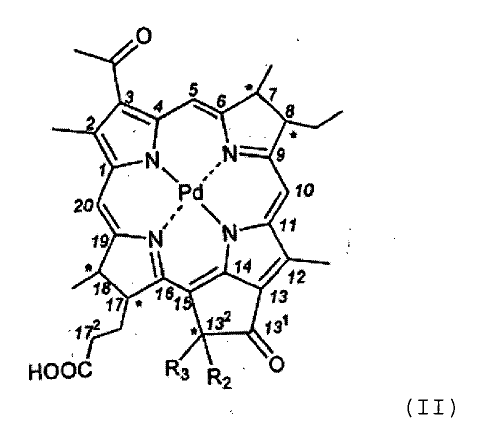 dans laquelle R₂ est un atome d'hydrogène, un groupe hydroxyle ou un groupe -COOR₄ dans lequel R₄ est un atome d'hydrogène, un groupe alkyle de 1 à 12 atomes de carbone ou un groupe cycloalkyle de 3 à 12 atomes de carbone, et R₃ est un atome d'hydrogène, un groupe hydroxyle ou un groupe alkyle ou alcoxy de 1 à 12 atomes de carbone. L'invention s'étend aussi aux formulations comprenant les sels de métaux alcalins ou alcalino-terreux des composés de formule générale (II) ci-dessus, en particulier les sels non solubles.

Dans la formule (II) ci-dessus, R₂ est de préférence un groupe -COO-alkyle où la partie alkyle, linéaire ou ramifiée, comporte 1 à 6 atomes de carbone, plus particulièrement un groupe -COOCH₃, et R₃ est un atome d'hydrogène.

Ainsi, la formulation de l'invention s'applique tout particulièrement au palladium-bactériophéophorbide a, ou Pd Bphéide-a, de formule (II) ci-dessus où R₂ est groupe acétoxy et R₃ un atome d'hydrogène, représenté par la formule générale (III) suivante :

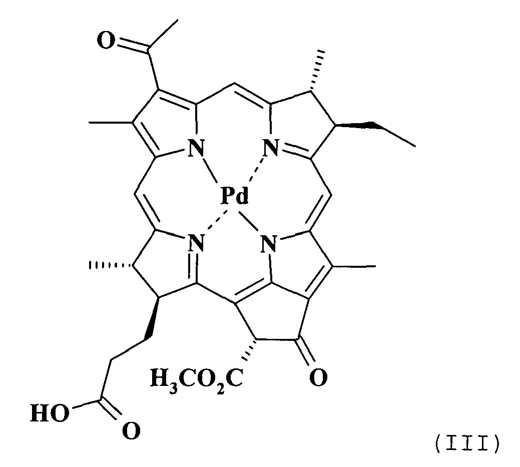

La présente invention a tout particulièrement pour objet une formulation pour administration par injection comprenant une substance photosensibilisante, utilisable en thérapie photodynamique ou en diagnostic, représentée par la formule générale (III) ci-dessus, réduisant l'agrégation de la substance photosensibilisante et limitant les réactions allergiques chez les patients à qui elle est administrée.

Enfin, l'invention a pour objet un procédé de préparation d'une formulation injectable comprenant une substance photosensibilisante de formule générale (II) ou (III) ci-dessus.

Les composés photosensibilisants représentés par les formules générales (II) et (III), ainsi que leurs sels, peuvent être préparés par le procédé décrit dans le brevet EP 1.137.411 précité.

Si certains sels des composés ci-dessus, tels que le sel de sodium, présentent une solubilité satisfaisante dans les solvants usuels tels que les alcools, par contre leur stabilité dans le temps est très limitée, ce qui interdit en pratique leur utilisation dans des compositions pharmaceutiques.

Les études effectuées suivant la présente invention ont montré que les effets secondaires observés avec les formulations classiques suivant les techniques connues rappelées ci-dessus, pouvaient être limités, et même évités, en utilisant une formulation conforme à la présente invention où la substance photosensibilisante est associée à un agent tensioactif en combinaison avec du mannitol, de préférence le d-mannitol, de manière à obtenir un taux d'agrégat de la substance photosensibilisante inférieur à 20%, c'est-à-dire un rapport en poids agrégat / monomère inférieur à 20/80, et de préférence inférieur à 15/85. Le mannitol joue ici le rôle d'agent augmentant l'osmolarité, c'est-à-dire le nombre de moles de soluté par masse de solvant, à la place d'un sel inorganique tel que le chlorure de sodium. Le mannitol permet d'ajuster la pression osmotique de la formulation à celle du sang, et n'augmente pas la force ionique de la solution. Il est donc plus efficace qu'un agent classique comme le chlorure de sodium qui a tendance à augmenter la force ionique et par conséquent à favoriser l'agrégation.

En réduisant aussi fortement le taux d'agrégats par rapport au taux de monomère de substance photosensibilisante dans la formulation, on améliore sensiblement l'efficacité thérapeutique comme l'ont montré les tests comparatifs effectués avec une formulation de l'invention comparée à une formulation conforme à la demande WO 03009842. De plus, le faible taux d'agrégats procure une meilleure sécurité d'utilisation de la formulation et limite certains effets secondaires, tels qu'un phénomène d'hypotension etun effet néfaste transitoire sur le foie, qui sont souvent observés avec les formulations connues.

Ainsi les études ont montré qu'un taux élevé de monomère est plus important qu'une forte concentration pour l'utilisation pharmaceutique car l'activité est liée au monomère tandis que les agrégats entraînent des effets secondaires comme indiqué ci-dessus. Ainsi, il est important que les formulations utilisables en thérapeutique présentent de manière fiable un taux d'agrégation toujours inférieur à 20%. Cet objectif est atteint par la formulation suivant la présente invention.

L'agent tensioactif utilisé dans la formulation de l'invention permet de faciliter la solubilisation de l'agent photosensibilisant. Il est choisi de préférence parmi le Cremophor EL®, le Cremophor ELP® et le Polysorbate 20®. Le Cremophor est un agent tensioactif à base d'huile de ricin polyoxyéthylénée, et le Polysorbate est un ester éthoxylé de sorbitan couramment utilisé comme émulsionnant et tensioactif non ionique.

Le solvant utilisé dans la formulation de l'invention est avantageusement un alcool tel que le méthanol ou l'éthanol. On utilise de préférence un alcool répondant aux critères de pureté de la Pharmacopée. Le solvant et le tensioactif sont utilisés dans un rapport en volume compris entre 1/1 et 1/10, et de préférence entre 1/2 et 1/5.

Suivant une forme avantageuse de réalisation de l'invention, la formulation comprend en outre un tampon permettant de procurer un pH supérieur à 7, et de préférence supérieur à 8. Le tampon utilisable dans l'invention est de préférence un mélange constitué par une base telle que la trométhamine (base Tris ou tris(hydroxyméthyl)aminométhane) ou la soude, et du mannitol.

La concentration en substance photosensibilisante de formule (II) ou (III), et en particulier la concentration de Pd-Bphéide-a de formule générale (III), est de préférence comprise entre 0,1 mg/ml et 1,5 mg/ml. Une concentration supérieure à 1,5 mg/ml peut entraîner une solubilisation incomplète de la substance solubilisante, une forte agrégation et une diminution sensible du taux de monomère. Une concentration inférieure à 0,1 mg/ml est généralement insuffisante pour procurer une efficacité satisfaisante en thérapie photodynamique, mais elle peut être envisagée dans des applications de diagnostic.

Ainsi une formulation suivant la présente invention comprend avantageusement le Pd Bphéide-a comme agent photosensibilisant en une concentration comprise entre 0,5 et 1,5 mg/ml ainsi qu'un tensioactif tel que le Cremophor de concentration comprise entre 20 et 80 mg/ml et du mannitol en concentration comprise entre 20 et 80 mg/ml.

La formulation suivant la présente invention possède une bonne stabilité dans le temps qui peut être encore sensiblement améliorée par une technique de lyophilisation. Le mannitol utilisé dans la formulation de la présente invention présente l'avantage de pouvoir être lyophilisé et de favoriser la lyophilisation en formant un produit de bonne stabilité dans le temps.

Le procédé de préparation d'une formulation injectable comprenant une substance photosensibilisante de formule générale (II) ou (III) ci-dessus comprend les étapes décrites ci-après.

Dans une première étape, on dissout l'agent photosensibilisant dans un mélange du solvant et du tensioactif, sous agitation, jusqu'à obtenir une solution concentrée homogène. Cette dissolution est réalisée de préférence à chaud, en opérant à une température comprise entre 20 et 40°C sous atmosphère inerte. Dans une deuxième étape on ajuste le pH à une valeur supérieure à 7 en ajoutant la base additionnée de mannitol. Dans une troisième étape optionnelle, on procède à une lyophilisation. La lyophilisation présente l'avantage de ne pas éliminer le tensioactif et le mannitol qui sont maintenus à l'état solide tandis que la majeure partie de l'alcool est éliminée. La solution injectable peut être reconstituée à partir de la composition lyophilisée par addition d'eau stérile de qualité pharmaceutique pour injection.

L'un des avantages de la présente invention est que les deux ou trois étapes du procédé ci-dessus peuvent être réalisées dans le même ballon, ou dans le même réacteur, ce qui facilite la mise en oeuvre, en particulier sur le plan industriel.

Les exemples suivants illustrent l'invention plus en détail sans en limiter la portée. Dans ces exemples, l'abréviation "WST 09" désigne le Pd Bphéide-a de formule générale (III) utilisable comme substance photosensibilisante. Sauf indication contraire, tous les pourcentages et parties sont exprimés en poids.

### Exemple 1

Dans un ballon de 250 ml, on ajoute successivement : WST-09 (1 g), Cremophor EL (73,5 g) et de l'éthanol (70 ml). Le Cremophor EL (BASF) est disponible auprès de la société Sigma (C-5135). Le mélange est maintenu sous agitation au moyen d'un agitateur magnétique, sous atmosphère d'argon, pendant 2 heures. Le concentré est réparti entre deux fioles (1,40 ml de concentré dans chaque fiole contenant 10,0 mg de WST-09), que l'on scelle sous argon.

Une solution de tampon de dilution est préparée comme suit: de la trométhamine (1,74 g) et du mannitol (48,2 g) sont ajoutés à un ballon d'1 litre. Le ballon est complété à 1 litre par de l'eau distillée et la solution est titrée à pH 8,6 avec HCl 1M.

Le concentré est ensuite dilué : une fiole de 10,0 mg de WST-09 est diluée avec 8,6 ml du tampon de dilution ci-dessus et on mélange le tout pendant 10 minutes jusqu'à apparition d'une solution rouge clair de 1 mg/ml de WST-09.

La teneur en monomère est de 92%.

### Exemple 2

Dans un ballon de 250 ml, on ajoute successivement : WST-09 (1 g), Cremophor EL (73,5 g) et de l'éthanol (30 ml). Le mélange est maintenu sous agitation au moyen d'un agitateur magnétique, sous atmosphère d'argon, pendant 18 heures. Le concentré est réparti entre deux fioles (0,98 g de concentré dans chaque fiole contenant 10,0 mg de WST-09), que l'on scelle sous argon.

Une solution de tampon de dilution est préparée comme suit: de la trométhamine (1,66 g) et du mannitol (48,3 g) sont ajoutés à un ballon d'1 litre. Le ballon est complété à 1 litre par de l'eau distillée et la solution est titrée à pH 8,4 avec HCl 1M.

Le concentré est ensuite dilué : à une fiole de 10,0 mg de WST-09 on ajoute 9 ml du tampon de dilution ci-dessus et on mélange le tout pendant 10 minutes jusqu'à obtenir une solution rouge clair de 1 mg/ml de WST-09.

La teneur en monomère est de 97%.

### Exemple 3

Dans un ballon de 100 ml, on introduit : WST-09 (40 mg), Polysorbate 20 (2,2 g) et de l'éthanol (1,2 ml). Le mélange est maintenu sous agitation au moyen d'un agitateur magnétique, sous atmosphère d'argon, pendant 1 heure. Le concentré est dilué avec 12,9 ml de tampon à 4,8% de mannitol et 0,15% de trométhamine, puis à nouveau par 23,9 ml d'une solution à 4,8% de mannitol. La formulation diluée est mélangée pendant 25 minutes.

La teneur en monomère est de 97%.

### Exemple 4

Dans un ballon de 100 ml, on introduit : WST-09 (40 mg), Solutol HS-15 (2,2 g) et de l'éthanol (1,2 ml). Le mélange est maintenu sous agitation au moyen d'un agitateur magnétique, sous atmosphère d'argon, pendant 1 heure. Le concentré est dilué avec 12,9 ml de tampon à 4,8% de mannitol et 0,15% de trométhamine, puis à nouveau par 23,9 ml d'une solution à 4,8% de mannitol. La formulation diluée est mélangée pendant 25 minutes.

La teneur en monomère est de 85%.

### Exemple 5

Dans un ballon de 100 ml, on introduit : WST-09 (15,8 mg), Cremophor EL (1,40 g) et de l'éthanol (0,58 ml). Le mélange est maintenu sous agitation au moyen d'un agitateur magnétique, sous atmosphère d'argon, pendant 30 minutes, puis le concentré est dilué avec 6,5 ml de tampon à 5% de mannitol et 0,0042 M de NaOH, puis à nouveau par 7,7 ml d'une solution à 5% de mannitol. La formulation diluée est mélangée pendant 10 minutes.

La teneur en monomère est de 93%.

### Exemple 6

Dans un réacteur de 10 litres sur système à bain d'eau, on ajoute, à 30°C, du Cremophor ELP (420 g préchauffés à 37°C) et de l'éthanol (240 ml). Le Cremophor ELP est obtenu de la société BASF. Les deux liquides sont mélangés pendant 5 minutes pour obtenir une phase homogène. On ajoute alors 8,56 g de WST-09, et le concentré formé est maintenu sous agitation pendant 1 heure à 30°C.

Le réacteur est ensuite refroidi à 25°C, et on ajoute 2 litres d'un tampon contenant 0,15% de trométhamine et 4,8% de mannitol. La formulation est ensuite mélangée sous agitation pendant 25 minutes à 25°C, puis on ajoute 4,5 litres de solution à 4,8% de mannitol, et on maintient sous agitation pendant encore 10 minutes. Le pH mesuré est de 8,19.

On ajoute encore 100 ml de tampon trométhamine - mannitol décrit ci-dessus pour porter le pH à 8,3, puis 760 ml de solution à 4,8% de mannitol pour porter au volume voulu, et le mélange est maintenu sous agitation pendant encore 10 minutes. Le pH final est de 8,33. La formulation est filtrée sur filtre Pall Flourodyne 0.2 µM 200 cm² en utilisant une pompe péristaltique. La solution filtrée est répartie en aliquotes de 50 ml dans des bouteilles de lyophilisation ambrée munies de bouchons butyle, puis on lyophilise.

Les solutions lyophilisées sont reconstituées en opérant de la manière suivante. Une bouteille de 50 mg de WST-09 est reconstituée avec 46,1 ml d'eau et mélangée doucement pendant 15 minutes.

La teneur en monomère est de 86%.

### Exemple 7

Dans un ballon de 250 ml, on introduit : WST-09 (1 g), Cremophor EL (73,5 g) et de l'éthanol (30 ml). Le mélange est maintenu sous agitation au moyen d'un agitateur magnétique, sous atmosphère d'argon, à température ambiante pendant 40 minutes. Le concentré est transféré dans un réacteur de 1 litre et on ajoute 315 ml de tampon à 0,15% de trométhamine dans 4,9% de mannitol, et la solution est maintenue sous agitation pendant 5 minutes à 25°C. Puis on ajoute à nouveau 585 ml d'une solution à 5% de mannitol et on maintient sous agitation pendant encore 20 minutes. La formulation de couleur rouge clair qui est obtenue est filtrée comme dans l'Exemple 6 ci-dessus, et répartie en aliquotes de 10 ml. La formulation est ensuite lyophilisée.

La solution lyophilisée est reconstituée en opérant de la manière suivante. Une fiole de 10 mg de WST-09 est reconstituée avec 9 ml d'eau et mélangée doucement pendant 15 minutes.

La teneur en monomère est de 83%.

### Exemple 8

Dans un ballon de 100 ml, on introduit : WST-09 (40 mg), Polysorbate 20 (2,2 g) et de l'éthanol (1,2 ml). Le mélange est maintenu sous agitation au moyen d'un agitateur magnétique, sous atmosphère d'argon, pendant 1 heure. Le concentré est dilué avec 12,9 ml de tampon à 4,8% de mannitol et 0,15% de trométhamine, puis à nouveau par 23,9 ml d'une solution à 4,8% de mannitol. La formulation diluée est mélangée pendant 25 minutes, puis filtrée comme indiqué dans l'Exemple 6. Le filtrat est réparti en aliquotes de 10 ml et lyophilisé.

La solution lyophilisée est reconstituée en opérant de la manière suivante. Une fiole de 10 mg de WST-09 est reconstituée avec 9 ml d'eau et mélangée doucement pendant 15 minutes.

La teneur en monomère est de 88%.

### Exemple 9

Dans un ballon de 100 ml, on introduit : WST-09 (40 mg), Solutol HS15 (2,2 g) et de l'éthanol (1,2 ml). Le mélange est maintenu sous agitation au moyen d'un agitateur magnétique, sous atmosphère d'argon, pendant 1 heure. Le concentré est dilué avec 12,9 ml de tampon à 4,8% de mannitol et 0,15% de trométhamine, puis à nouveau par 23,9 ml d'une solution à 4,8% de mannitol. La formulation diluée est mélangée pendant encore 25 minutes, puis filtrée comme indiqué dans l'Exemple 6. Le filtrat est réparti en aliquotes de 10 ml et lyophilisé.

La solution lyophilisée est reconstituée en opérant de la manière suivante. Une fiole de 10 mg de WST-09 est reconstituée avec 9 ml d'eau et mélangée doucement pendant 15 minutes.

La teneur en monomère est de 84%.

### Exemple 10

Dans un ballon de 100 ml, on introduit : WST-09 (15,8 mg), Cremophor EL (1,4 g) et de l'éthanol (0,58 ml). Le mélange est maintenu sous agitation au moyen d'un agitateur magnétique, sous atmosphère d'argon, pendant 30 minutes. Le concentré est dilué avec 6,5 ml de tampon à 5% de mannitol et 0,0042 M de NaOH, puis à nouveau par 7,7 ml d'une solution à 5% de mannitol. La formulation maintenue sous agitation pendant encore 10 minutes, puis filtrée comme indiqué dans l'Exemple 6. La solution est répartie en aliquotes de 1 ml et lyophilisée.

La solution lyophilisée est reconstituée en opérant de la manière suivante. Une fiole de 1,0 mg de WST-09 est reconstituée avec 0,9 ml d'eau, et mélangée doucement pendant 15 minutes.

La teneur en monomère est de 86%.

### Exemple 11

Une formulation injectable utilisant la formulation concentrée de l'Exemple 6 est réalisée suivant les techniques classiques à partir de l'agent photosensibilisant WST 09 et des excipients dans la composition centésimale indiquée ci-dessous.

| | |
|---|---|
| WST 09 | 0,10 % (p/v) |
| Cremophor ELP | 5,00 % (v/v) |
| Base Tris | 0,046% (p/v) |
| Mannitol | 4,42 % (p/v) |

Cette formulation est utilisée dans les tests décrits dans les exemples suivants.

### Exemple 12

Test d'efficacité chez la souris Nude L'efficacité chez la souris Nude CD-1 a été évaluée par comparaison avec une formulation connue conforme à celle décrite dans la demande de brevet WO 030009842 comprenant le même agent photosensibilisant (WST 09) et ayant la composition suivante :

| | |
|---|---|
| WST 09 | 0,25 % (p/v) |
| Cremophor ELP | 5,00 % (v/v) |
| Alcool éthylique | 4,00 % (v/p) |
| Alcool benzylique | 1, 00 % (v/p) |
| NaOH 0,0084M | qs pH 8,7 |
| Acide citrique | qs pH 7,1 |
| Eau | qsp 50 ml |

Le test est effectué sur des souris Nude CD-1 mâles (28-32 g) préalablement anesthésiées par injection ip de 40 µl de mélange 85/15 de Ketamine (100 mg/ml, Rhone-Merieux, France) et de Xylazine (2%, Vitamed, Israel).

Les résultats montrent que la formulation de l'invention est environ deux fois plus active que la formulation de comparaison ci-dessus. Dans ce modèle, la formulation de comparaison est inactive à la dose de 2 mg/kg de poids, partiellement active à 4 mg/kg active à 8 mg/kg, tandis que la formulation de l'invention est partiellement active dès 2 mg/kg et active à 4 mg/kg. De plus, on constate que la formulation de l'invention à 8 mg/kg provoque la mort de l'animal par surdose.

Ces résultats sont corrélés à la teneur en monomère dans chaque formulation qui est de 14% dans la formulation de l'invention et proche de 50% dans la formulation connue.

### Exemple 14

### Essais chez le chien

L'objectif de l'étude était de confirmer les résultats obtenus chez la souris nude (exemple 12) en comparant l'efficacité de la formulation de l'invention (conforme à l'Exemple 12) avec une formulation connue (conforme à WO 2003009842) sur prostate normale chez le chien. Le photosensibilisant a été préparé à une concentration de 0.9 mg/ml à partir du lyophilisat par addition d'eau ppi. Le ratio monomères/ agrégats était de 88/12. Le traitement PDT a été réalisé par illumination interstitielle via des fibres émettrices placées au centre de chaque lobe prostatique et reliées à un laser à 763 nm. La gamme de doses testées a été de 0.25 à 1 mg/kg pour des énergies de 0 à 200 J/cm. Un total de 28 animaux a été inclus. La taille des lésions induites a été évaluée par analyse macroscopique une semaine après la procédure. La taille des lésions est dose- et énergie-dépendante pour atteindre un diamètre d'approximativement 23 mm à 1 mg/kg et 100 J/cm et 18 mm à 1 mg/kg et 50 J/cm. Ces tailles de lésions prostatiques étaient obtenues avec 2 mg/kg de la formulation connue. L'analyse histologique est identique avec les 2 formulations. En conclusion la formulation de l'invention est environ deux fois plus active que la formulation connue.

### Pharmacocinétique

Les résultats obtenus dans l'essai de pharmacodynamie décrit ci-dessus sont confirmés par les études de pharmacocinétique menées chez les animaux recevant la formulation de l'invention.

Les résultats sont rassemblés dans le tableau ci-dessous. La comparaison montre que pour une même dose injectée, la concentration plasmatique est supérieure d'un facteur 2 pour la formulation (LC-45) conforme à l'invention par comparaison avec une formulation connue (TOOKAD).

| | | | | | |
|---|---|---|---|---|---|
| Espèce Méthode | **Chien Beagle** | | | | |
| d'administration | Intraveineuse (infusion 10 min) | | | | |
| Formulation | TOOKAD | LC-45 | | | |
| Gender (M/F) / Nb d'animaux | M/4 | M/5 | M/5 | M/5 | M/3 |
| Dose (mg.kg⁻¹) | 2 | 0,25 | 0,5 | 0,75 | 1 |
| Echantillon | Plasma | Plasma | Plasma | Plasma | Plasma |
| Essai | HPLC/UV | HPLC/UV | HPLC/UV | HPLC/UV | HPLC/UV |
| | | | | | |
| paramètres de PK | | | | | |
| Cmax (ng.mL⁻¹) (Moyen ± SD) | 6033.35 ± 2689.61 | 1151.14 ± 167.20 | 2985.76 ± 288.06 | 4527.08 ± 904.01 | 6640.78 ± 2009.21 |
| Tmax (h) (Median) | 0,15 | 0,17 | 0,17 | 0,17 | 0,13 |
| AUCₜ (ng.h.mL⁻¹) (Moyen ± SD) | 1963.78 ± 590.78 | 401.53 ± 116.41 | 1010.72 ± 147.39 | 1336.96 ± 373.95 | 1960.36 ± 729.61 |
| AUC_{inf} (ng.h.mL⁻¹) (Moyen ± SD) | NC | 464.15 ± 145.67 | 1095.84 ± 183.25 | 1442.88 ± 424.96 | 2055.90 ± 739.81 |
| Kel (Moyen ± SD) | NC | 2.8280 ± 1.7412 | 2.2788 ± 0.7770 | 3.1713 ± 0.9855 | 2.1432 ± 1.0826 |
| t_{1/} (Moyen ± SD) | NC | 0.32 ± 0.16 | 0.37 ± 0.23 | 0.23 ± 0.05 | 0.43 ± 0.30 |

| | | | | | |
|---|---|---|---|---|---|
| NC : non calculable (données insuffisantes) | | | | | |

### Exemple 15

### Essais chez le singe Cynomolgus

Afin de confirmer l'hypothèse du rôle des agrégats dans l'induction des chutes de la pression artérielle, l'étude des effets cardiovasculaires de la formulation de l'invention a été réalisée chez le singe anesthésié.

5 singes Cynomolgus ont reçus les doses de 0, 3, 4, 5 mg/kg pour des vitesses d'injection de 4, 5 et 8 ml/min. Chaque série de traitements était séparée par un wash-out de 2 semaines. Un électrocardiogramme est réalisé avant et après l'injection de la formulation.

Dans ces conditions, la formulation de l'invention aux doses de 3, 4, 5 mg/kg à 4 ml/min et à la dose de 4 mg/kg à 8 ml/min n'induit pas de changement significatif de la pression artérielle, du rythme cardiaque et des temps de conduction cardiaque. Par contre une dose de 5 mg/kg et 4 ml/min induit une chute de la pression artérielle (30% par rapport à la pression artérielle moyenne basale) chez trois animaux sur cinq 0.25 heures après la fin de l'injection. A 4 mg/kg et 8 ml/min seul un animal a présenté un léger épisode hypotenseur. En conclusion, 4 mg/kg et 8 ml/min est considéré comme la dose sans effet (NOEL).

Le même design d'étude a été réalisé pour évaluer les effets cardiovasculaires de la formulation connue (TOOKAD) de comparaison mentionnée ci-dessus chez le singe conscient. Les doses de 2 (2, 3, 4 ml/min) et 5 mg/kg (2 ml/min) ont été testées. La dose de 2 mg/kg (2, 3, 4 ml/min) n'a induit ni effet sur la pression artérielle ni variation de l'ECG. Par contre une chute de la pression artérielle a été observée à la dose de 5 mg/kg (2 ml/min). Lors d'une étude préliminaire avec la formulation connue de comparaison, chez des singes anesthésiés, la dose de 4 mg/kg (4 ml/min) a été considérée comme inductrice d'hypotension alors que la dose de 2 mg/kg (5 ml/min) était sans effet notable.

En conclusion et dans les conditions testées, la formulation de l'invention a une dose sans effet et, en particulier sans effet hypotenseur, deux fois plus élevée que la formulation connue de comparaison.

## Revendications

1. Formulation pour administration par injection, comprenant une substance photosensibilisante utilisable en thérapie photodynamique ou en diagnostic représentée par la formule générale (II) suivante : 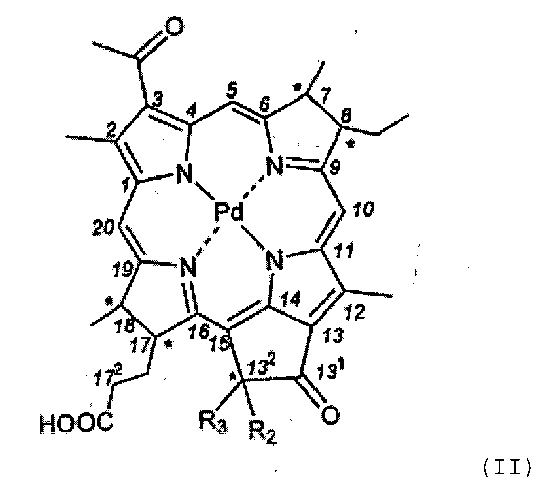 dans laquelle R₂ est un atome d'hydrogène, un groupe hydroxyle ou un groupe -COOR₄ dans lequel R₄ est un atome d'hydrogène, un groupe alkyle de 1 à 12 atomes de carbone ou un groupe cycloalkyle de 3 à 12 atomes de carbone, et R₃ est un atome d'hydrogène, un groupe hydroxyle ou un groupe alkyle ou alcoxy de 1 à 12 atomes de carbone,ou un de ses sels de métaux alcalins ou alcalino-terreux,
en combinaison avec un agent tensioactif et du mannitol.

2. Formulation selon la revendication 1, **caractérisée en ce que** la substance photosensibilisante est représentée par la formule générale (III) suivante :

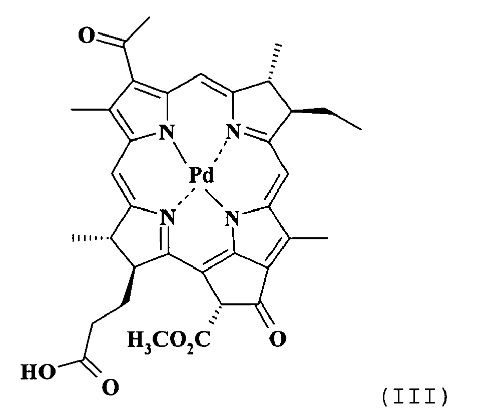

3. Formulation selon la revendication 2, **caractérisée en ce que** la substance photosensibilisante est présente sous forme de monomère et d'agrégats dans un rapport en poids agrégat / monomère inférieur à 20/80.

4. Formulation selon la revendication 1, **caractérisée en ce que** la concentration de mannitol est comprise entre 20 et 80 mg/ml.

5. Formulation selon la revendication 1, **caractérisée en ce que** la concentration de l'agent tensioactif est comprise entre 20 et 80 mg/ml.

6. Formulation selon l'une quelconque des revendications 1 et 5, **caractérisée en ce que** l'agent tensioactif est choisi parmi le Cremophor EL^{®}, le Cremophor ELP^{®}, le Polysorbate 20.

7. Formulation selon l'une quelconque des revendications précédentes, **caractérisée en ce qu'**elle comprend en outre un tampon basique.

8. Formulation selon la revendication 7, **caractérisée en ce que** la base est choisie parmi la trométhamine (base Tris) et la soude.

9. Formulation selon l'une quelconque des revendications précédentes, **caractérisée en ce** la concentration en substance photosensibilisante est comprise entre 0,1 mg/ml et 1,5 mg/ml.

10. Procédé de préparation d'une solution injectable utilisable en thérapie photodynamique ou en diagnostic comprenant une substance photosensibilisante suivant la revendication 1, **caractérisé en ce qu'**il comprend les étapes suivantes :
dans une première étape, on dissout l'agent photosensibilisant dans un mélange du solvant et du tensioactif, sous agitation, jusqu'à obtenir une solution concentrée homogène,
dans une deuxième étape on ajuste le pH à une valeur supérieure à 7 en ajoutant un tampon additionné de mannitol.

11. Procédé selon la revendication 10, **caractérisé en ce qu'**il comprend une troisième étape optionnelle de lyophilisation.
